# EUROPÄISCHE PATENTSCHRIFT

(11) **EP 2 340 071 B1**
(45) Veröffentlichungstag und Bekanntmachung des Hinweises auf die Patenterteilung: **28.01.2015**
(21) Anmeldenummer: 09778763.4
(22) Anmeldetag: 29.09.2009
(51) Int. Cl.: A61M 5/178, A61M 5/30, A61J 1/20

(54) **INJEKTOR UND ZWEI-KAMMER-SYSTEM MIT ABDICHTENDEM BEHÄLTERADAPTER**
INJECTOR AND TWO-CHAMBER SYSTEM HAVING SEALING CONTAINER ADAPTER
DISPOSITIF D'INJECTION ET SYSTÈME À DEUX CHAMBRES AVEC ADAPTATEUR DE RÉCIPIENT ÉTANCHÉIFIANT

(30) Priorität: 31.10.2008 DE 102008054128
(43) Veröffentlichungstag der Anmeldung: 06.07.2011
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Siemund, Volker
(86) Internationale Anmeldenummer: PCT/EP2009/006996
(87) Internationale Veröffentlichungsnummer: WO 2010/049045

(56) Entgegenhaltungen:
- EP-A2- 0 882 441
- WO-A2-00/23133
- DE-T2- 69 003 805

## Beschreibung

Die Erfindung betrifft einen Einweginjektor und ein Zwei-Kammer-System, wobei zumindest eine erste Kammer Teil einer im Einweginjektor aufnehmbaren Zylinder-Kolben-Einheit ist und wobei die zweite Kammer Teil eines zumindest zeitweise mittels eines Stopfens verschlossenen und in einem lösbar am Einweginjektor gelagerten Behälteradapter eingesetzten Behälters mit mindestens einer Öffnung ist.

Ein derartiger Einweginjektor und ein Zwei-Kammer-System sind beispielsweise aus der nachveröffentlichten DE 10 2008 003 105 A1 bekannt. Ein in den Behälteradapter eingesetzter Doppeladapter schiebt den Stopfen aus der Öffnung, so dass der Stopfen in den Behälter fällt.

Ein weiterer aus dem Stand der Technik bekannter Einweginjektor ist im Dokument WO 00/23133 A offenbart. Der Oberbegriff des Anspruchs 1 ist auf diesem Dokument basiert.

Der vorliegenden Erfindung liegt daher die Problemstellung zugrunde, einen Einweginjektor und ein Zwei-Kammer-System mit einer verringerten Anzahl von Bauteilen zu entwickeln.

Diese Problemstellung wird mit den Merkmalen des Hauptanspruches gelöst. Dazu sind der Stopfen und der Behälteradapter miteinander unlösbar verrastbar. Beim Einsetzen des Behälters verschließt der Behälteradapter die Öffnung unter Verdrängung des Stopfens. Der Behälteradapter verbindet nach dem Einsetzen des Behälters den Zylinderinnenraum der Zylinder-Kolben-Einheit mit dem Innenraum des Behälters.

Weitere Einzelheiten der Erfindung ergeben sich aus den Unteransprüchen und der nachfolgenden Beschreibung schematisch dargestellter Ausführungsformen.
- Figur 1:: Einweginjektor und Zwei-Kammer-System;
- Figur 2:: Einweginjektor und teilweise eingeschobener Behälter;
- Figur 3:: Einweginjektor nach dem Einschieben des Behälters;
- Figur 4:: Einweginjektor nach dem Umpumpen;
- Figur 5:: Einweginjektor mit Zylinder-Kolben-Einheit vor dem Auslösen;
- Figur 6:: Figur 4 nach dem Auslösen;
- Figur 7:: Detail von Figur 1;
- Figur 8:: Detail von Figur 2;
- Figur 9:: Detail von Figur 3;
- Figur 10:: Detail eines Einweginjektors und Zwei-Kammer-System mit einer Zylinder-Kolben-Einheit;
- Figur 11:: Figur 10 beim Einschieben des Behälters;
- Figur 12:: Figur 10 nach dem Einschieben des Behälters;
- Figur 13:: Detail von Figur 12;
- Figur 14:: Behälteradapter mit Stopfen;
- Figur 15:: Behälter mit Verschlußeinsatz.

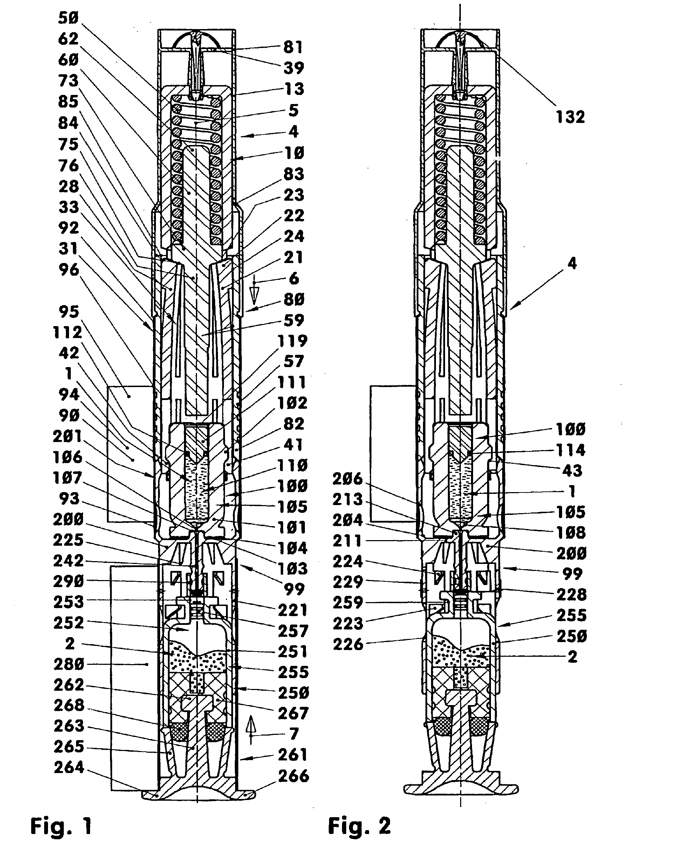

Die Figuren 1 - 5 zeigen einen Einweginjektor (4) und ein an diesen adaptiertes Zwei-Kammer-System (99). Die Figur 1 zeigt z.B. den Auslieferungszustand an den Anwender, in dem das Zwei-Kammer-System (99) in den Einweginjektor (4) integriert ist und der Einweginjektor (4) vorgespannt ist. Die erste Kammer (105) ist mit Lösemittel (1) und die zweite Kammern (255) mit Lyophilisat (2) z.B. partiell befüllt. Beide Kammern (105, 255) sind voneinander getrennt.

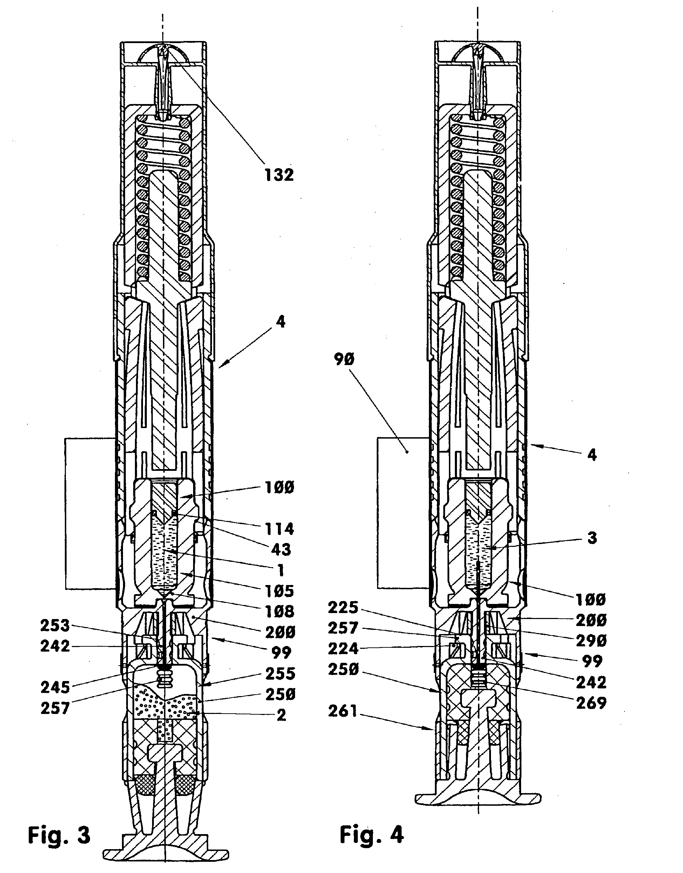

Die Figur 2 zeigt einen Zwischenzustand bei der Verbindung der beiden sterilen Kammern (105, 255). In der Figur 3 sind die beiden Kammern (105, 255) zur Herstellung einer Injektionslösung (3) miteinander verbunden.

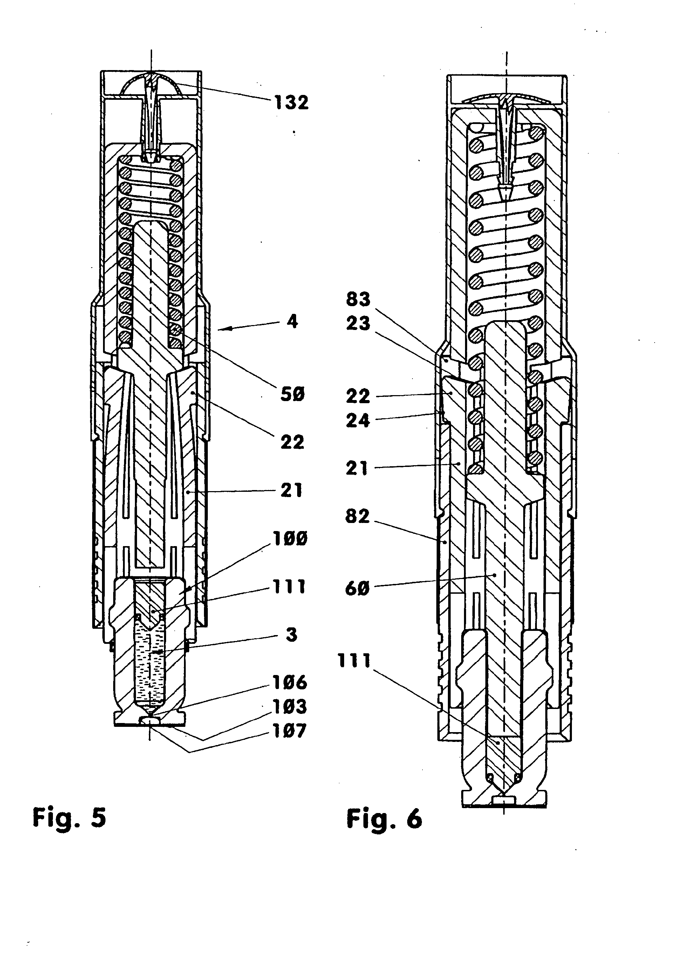

Die Figur 4 zeigt den Einweginjektor (4) und das Zwei-Kammer-System (99) nach der Herstellung der Injektionslösung (3) und die Figur 5 diesen Injektor (4) mit der injektorseitigen Kammer (105) vor dem Auslösen. In der Darstellung der Figur 6 ist der nadellose Einweginjektor (4) ausgelöst und die Injektionslösung (3) herausgespritzt.

Der in den Figuren 1 - 6 dargestellte Einweginjektor (4) umfasst ein Gehäuse (10), einen Kolbenbetätigungsstempel (60) und eine Schraubendruckfeder (50) als Federenergiespeicher. Zudem sind am Gehäuse (10) eine Auslöseeinheit (80) mit einem Auslöseelement (82) und in den Figuren 1 - 4 ein Sicherungselement (90) angeordnet.

Das Gehäuse (10) ist ein einteiliger, topfförmiger, unten offener Hohlkörper mit obenliegendem Boden (39). Es wird z.B. aus einem glasfaserverstärkten Polyamid durch Spritzgießen gefertigt. Das Gehäuse (10) hat eine weitgehend rohrförmige Gestalt und ist in zwei Funktionsbereiche aufgeteilt, das sind zum einen der obere Mantelbereich (31) und zum anderen der untere Fixierbereich (41).

Im Mantelbereich (31) hat das Gehäuse (10) z.B. zwei einander gegenüberliegende fensterartige Durchbrüche (33). Am unteren Rand des einzelnen Durchbruchs (33) ist jeweils ein Druckstab (21) als elastischer Biegebalken angeformt. Die Anformstelle für die Druckstäbe (21) liegt knapp oberhalb des Fixierbereichs (41). Zur Ausbildung des jeweiligen Druckstabs (21) befindet sich im unteren Bereich des Mantelabschnitts (31) ein schmaler, zumindest annähernd u-förmiger Spalt, der den einzelnen Druckstab (21) seitlich und oben umgibt.

Der Druckstab (21) hat beispielsweise auf 80% seiner Länge die Wandstärke und die Krümmung der Wandung des Gehäuses (10). Dieser Bereich hat unter anderem auch die Funktion eines federelastischen Biegebalkens (28). Er hat einen sichelförmigen Querschnitt.

Ggf. kann ein Teil dieses Biegebalkens (28) auch mit einem rechteckigen Querschnitt ausgestattet sein, um bei der Nutzung auftretende Biegespannungen im Biegebalkenrandbereich zu reduzieren.

Bei Injektoren, bei denen der Kolbenbetätigungsstempel (60) im Gehäuse (10) - zumindest abschnittsweise - mit geringem Spiel geradgeführt ist und der Kolbenbetätigungsstempel (60) eine ausreichende Biegefestigkeit aufweist, kann anstatt zweier oder mehrerer Druckstäbe (21) auch nur ein einziger Druckstab (21) verwendet werden.

Das hier obere freie Ende des einzelnen Druckstabs (21) wird durch den radial nach außen abstehenden Nocken (22) gebildet. Letzterer hat zumindest eine in Richtung der Mittellinie (5) orientierte Abstützfläche (23) und eine der Mittellinie (5) abgewandte Anlagefläche (24).

Die untere Hälfte des Gehäuses (10) ist von dem hülsenartigen Auslöseelement (82) umgeben. Dieses ist z.B. im Wesentlichen zylindrisch ausgebildet und beispielsweise aus AcrylnitrilButadien-Styrol-Copolymer (ABS) hergestellt. Das Auslöseelement (82) ist auf der radialen Außenfläche (13) des Gehäuses (10) längs verschiebbar gelagert. Es endet rückwärtig mit einer scharfen Kante (85), die Teil einer stirnseitigen Rücksprungflanke (84) des Auslöseelements (82) ist. Unterhalb der Kante (85) berühren nach Figur 1 die an die Druckstäbe (21) angeformten Nocken (22) mit ihren außen liegenden Anlageflächen (24) sichernd die Innenwandung (59) des Auslöseelements (82).

Beispielsweise in der Nähe der Kante (85) ist am Auslöseelement (82) eine Auslösekappe (81) befestigt, die das hintere Ende des Gehäuses (10) vollständig umgibt. Die Auslösekappe (81) umfasst eine umlaufende Aufweitung (83), in der beim Auslösen des Injektors die Nocken (22) aufgenommen werden, vgl. Figur 6. Anstelle dieser Aufweitung (83) können bei einem nichtrotationssymmetrischen Auslöseelement (82) pro Druckstab (21) auch partielle Aufweitungen oder nicht abgedeckte Öffnungen vorhanden sein. Oberhalb der Aufweitung (83) liegt die Auslösekappe (81) gleitfähig an der Außenwandung (13) des Gehäuses (10) an.

Der im Gehäuse (10) angeordnete Kolbenbetätigungsstempel (60) ist in zwei Bereiche aufgeteilt. Der untere Bereich ist der Kolbenschieber (76). Sein Durchmesser ist etwas kleiner als der Innendurchmesser des hinteren Bereichs des Zylinders (101) einer Zylinder-Kolben-Einheit (100). Die untere Stirnfläche des Kolbenschiebers (76) wirkt direkt auf den Kolben (111) dieser Zylinder-Kolben-Einheit (100).

Der obere Bereich des Kolbenbetätigungsstempels (60), der Stempelteller (73), ist eine flache, zumindest bereichsweise zylindrische Scheibe, deren Außendurchmesser einige Zehntel Millimeter kleiner ist als der Innendurchmesser des Gehäuses (10) im Mantelbereich (31). Die untere Stirnseite weist eine um den Kolbenschieber (76) herum angeordnete Bundfläche (75) auf. Sie hat die Form eines Kegelstumpfmantels, dessen Spitzenwinkel ca. 100 bis 140 Grad beträgt. Im dargestellten Ausführungsbeispiel hat die Bundfläche (75) einen Spitzenwinkel von 140 Grad. Die gedachte Spitze des Kegelstumpfmantels liegt auf der Mittellinie (5) im Bereich des Kolbenschiebers (76). Die Bundfläche (75) kann auch sphärisch gekrümmt sein.

Der Kolbenschieber (76) kann selbstverständlich auch als separates, vom Stempelteller (73) getrenntes Bauteil ausgeführt sein. Hierzu ist er dann an der Innenwandung des Gehäuses (10) geführt.

Zwischen dem Stempelteller (73) und dem oben liegenden Boden (39) des Gehäuses (10) sitzt vorgespannt die Schraubendruckfeder (50). Die Schraubendruckfeder (50) stützt sich am Boden (39) des Gehäuses (10) ab. Die Federkraft der Schraubendruckfeder (50) wird über den Stempelteller (73) auf die Druckstäbe (21) übertragen. Aufgrund der Neigung der Bundfläche (75) werden die Druckstäbe (21) keilgetriebeartig radial nach außen gedrängt. Die Auslösehülse (82) stützt diese Radialkraft dauerhaft ab.

Der Kolbenbetätigungsstempel (60) hat oberhalb des Stempeltellers (73) einen Führungszapfen (62). Letzterer führt die Schraubendruckfeder (50) oder wird durch diese geführt. Unterhalb des Stempeltellers (73) befindet sich zentral in der Verlängerung des Führungszapfens (62) der Kolbenschieber (76). Unterhalb des Mantelabschnitts (31) befindet sich der Fixierbereich (41) zur Aufnahme der einbaubaren Zylinder-Kolben-Einheit (100), die die erste Kammer (105) umfasst. Der Fixierbereich (41) umfasst z.B. acht parallel zur Mittellinie (5) ausgerichtete Federhaken (42). Die Federhaken (42) haben jeweils einen mindestens zweiflankigen Hintergriff (43) zur spielfreien Aufnahme der Zylinder-Kolben-Einheit (100). Die einander gegenüber liegenden Flanken des Hintergriffs (43) schließen einen Winkel von z.B. 90 Winkelgraden ein. Die Länge und die Federrate der Federhaken (42) sind so dimensioniert, dass die Zylinder-Kolben-Einheit (100) ohne plastische Verformung der Federhaken (42) eingebaut werden kann.

Die Zylinder-Kolben-Einheit (100) besteht im Ausführungsbeispiel aus einem mit Wasser für Injektionszwecke (1) oder einer Injektionslösung (3) befüllbaren, transparenten Zylinder (101). Das Wasser für Injektionszwecke (1) kann bereits Wirkstoffe enthalten. In der Darstellung der Figur 1 sitzt der Kolben (111) in der hinteren Position. Oberhalb des Kolbens (111) ist im Gehäuse (10) der Kolbenbetätigungsstempel (60) z.B. so angeordnet, dass er den Kolben (111) zwar nicht berührt, jedoch mit seinem unteren Ende z.B. im oberen Bereich des Zylinders (101) seitlich geführt wird.

Der Zylinder (101) ist z.B. ein klarsichtiger, dickwandiger Topf, dessen ggf. zylindrische Außenwandung einen beispielsweise umlaufenden Rastring (102) trägt, der an den Flanken des Hintergriffs (43) der Federhaken (42) formsteif anliegt. In der beispielsweise zylindrischen Bohrung des Zylinders (101) sitzt der stangenlose Kolben (111). Der Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. In der rückseitigen Stirnfläche des Kolbens (111) ist ggf. eine z.B. zylindrische Metallplatte eingelassen.

Im Zentrum der Bohrung des Zylinders (101), dessen Zylinderboden der Kontur der vorderen Kolbenstirnseite zumindest annähernd angepasst ist, befindet sich eine kurze zylindrische, düsenartige Bohrung (106). Ihr Durchmesser beträgt ca. 0,1 bis 0,5 Millimeter. Diese Bohrung (106) ist ein- bis fünfmal so lang wie ihr Durchmesser. Sie endet in einer zylindrischen Ausnehmung (107) der bodenseitigen, äußeren Stirnfläche (103) des Zylinders (101). Diese Stirnfläche (103) kann zur Erhöhung der Applikationssicherheit zusätzlich mit einem Klebering (104) versehen werden.

Der Zylinder (101) ist rückseitig mit einer Sterilfiltermembrane (119) steril verschlossen.

In den Einweginjektor (4) ist weiterhin ein Behälteradapter (200) eingesetzt. Dies ist ein büchsenartiges Bauteil, das z.B. die zweite Kammer (255) - diese umfasst hier einen als Zylinder-Kolben-Einheit (250) ausgebildeten Behälter (250) - in einem Behälterbereich (221) aufnimmt. Zugleich hat er einen hülsenförmigen Adapterbereich (201) mit dem er längsverschiebbar im Gehäuse (10) sitzt.

Der Behälteradapter (200) ist beispielsweise ein zumindest bereichsweise elastisch verformbares, ein- oder mehrkomponentiges Bauteil. Bei einem mehrkomponentigen Aufbau kann der Behälteradapter (200) Bereiche unterschiedlicher Steifigkeit und Elastizität haben. So kann z.B. der Adapterbereich (201) elastisch verformbar und der Behälterbereich (221) verformungssteif ausgebildet sein.

Der Adapterbereich (201) ist ein zylindrischer Becher, der zumindest das untere Fünftel des Zylinders (101) mit Abstand umgibt. Er hat zwei einander gegenüberliegende z.B. kreisrunde Fenster (206) und einen ringförmigen Absatz (204) auf dem Zwischenboden (211). Die Fenster (206) können entfallen, wenn das Behälteradaptermaterial transparent ist.

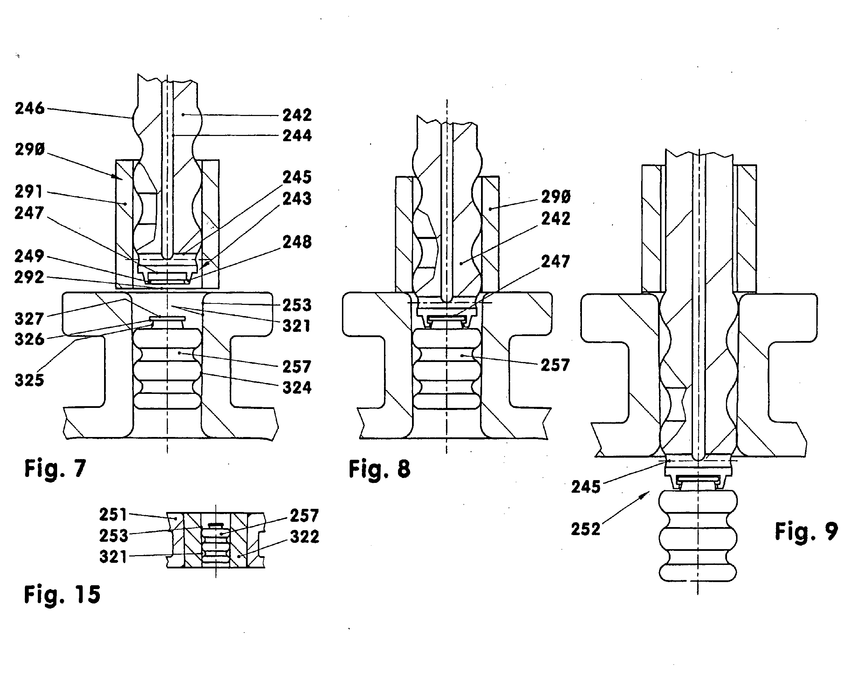

Zentral im Zwischenboden (211) hat der Behälteradapter (200) ein Übertragungsrohr (242), das den Adapterbereich (201) und den Behälterbereich (221) miteinander verbindet. Zur Zentrierung an der Ausnehmung (107) hat die dem Adapterbereich (201) zugewandte Fläche des Zwischenbodens (211) eine zentrale Erhebung (213). Der minimale Innendurchmesser des Übertragungsrohrs (242), der Durchmesser der Bohrung (244), entspricht mindestens dem Durchmesser der düsenartigen Bohrung (106). Der minimale Durchmesser der Bohrung (244) kann beispielsweise einen Millimeter betragen. Der Durchmesser der Bohrung (244) kann sich von beiden Stirnseiten zur Mitte hin oder von einer zur anderen Stirnseite hin z.B. kegelförmig verjüngen. Das Übertragungsrohr (242) hat beispielsweise einen maximalen Außendurchmesser von acht Millimetern. Es ist in der Darstellung der Figur 1 in dem in Richtung des Behälterbereichs (221) an den Zwischenboden (211) angrenzenden Bereich zylindrisch ausgebildet. Zum Rohrende (243), vgl. Figur 7, hin trägt das Übertragungsrohr (242) z.B. drei wulstartige, elastisch verformbare Ringe (246). Der Außendurchmesser dieser Ringe (246) ist im unverformten Zustand geringfügig größer als der Durchmesser der Öffnung (253) des Behälters (250), so dass das Übertragungsrohr (242) nach dem Einsetzen diese Öffnung (253) dicht verschließt.

In der Darstellung der Figuren 1 - 6 hat das Übertragungsrohr (242) unterhalb der Ringe (246) eine durchgehende Querbohrung (245), deren Durchmesser beispielsweise dem Durchmesser der als Sacklochbohrung ausgebildeten Längsbohrung (244) entspricht. Die Querbohrung (245) fluchtet mit zwei zumindest annähernd radial angeordneten Schieberausnehmungen (228) in der beispielsweise zylindrischen Seitenwand der Behälteraufnahme (221). Zumindest annähernd bedeutet hier, dass die Bohrungsmittellinie mit einer Radialen einen Winkel von bis zu 45 Grad einschließen kann. Diese Schieberausnehmungen (228) werden z.B. bei der Herstellung des Behälteradapters (200) von zwei Schiebern durchdrungen, die das Übertragungsrohr (242) halten und die Querbohrung (245) erzeugen. Die Schieberausnehmungen (228) sind beispielsweise mittels eines Ventilschlauchs (229) abgedeckt.

Das hier dargestellte Rohrende (243) weist eine z.B. zentrale Schiebefläche (247) auf, die von einem Rastring (248) umgeben ist. Der Rastring (248) hat beispielsweise eine umlaufende, nach innen orientierte Rastnase (249). Anstatt eines Rastrings (248) können an der Stirnseite des Übertragungsrohrs (242) z.B. drei jeweils um 120 Winkelgrad zueinander versetzte Rasthaken angeordnet sein. Die Schiebefläche (247) kann auch außerhalb des Rastrings (248) oder außerhalb der Rasthaken angeordnet sein. Auch kann die Stirnfläche des Rastrings oder eine durch die Stirnflächen von Rasthaken gebildete Ebene eine Schiebefläche bilden.

In dem in der Figur 1 und 7 dargestellten Ausführungsbeispiel sitzt auf der dem Behälterbereich (221) zugewandten Stirnseite des Übertragungsrohrs (242) festhaftend eine topfförmige Membrankappe (290). Diese umfasst einen z.B. zylinderförmigen, elastisch verformbaren Wandbereich (291) und eine als Membran (292) ausgebildete Stirnseite. Die Membran (292) kann auch an den Wandbereich (291) angeformt sein. Die Wandstärke des Wandbereichs (291) beträgt z.B. einen Millimeter.

Der Behälterbereich (221) des Behälteradapters (200) weist beispielsweise zwei Gruppen von Rastelementen (223, 224) auf, die unterschiedlich weit vom Zwischenboden (211) beabstandet sind. Das einzelne Rastelement (223, 224) ist beispielsweise ein dreieckförmiges nichtradial aus der Innenwandung des Behälterbereichs (221) hervorstehendes Element.

Im Behälterbereich (221) ist die Zylinder-Kolben-Einheit (250) angeordnet. Ihr Außendurchmesser ist nur geringfügig kleiner als der Innendurchmesser des Behälterbereichs (221).

Die Zylinder-Kolben-Einheit (250) hat einen Zylinder, der aus einem transparenten Rohr (251), z.B. einem Glas- oder Kunststoffrohr (COC), und einem elastischen Stopfen (257) gebildet wird. Der Stopfen (257) sitzt in der Darstellung der Figuren 1 und 2 sowie in den Detaildarstellungen der Figuren 7 und 8 in der Öffnung (253) des Behälters (250).

Die Öffnung (253) des z.B. flaschenartig ausgebildeten Behälters (250) kann zylindrisch oder konisch sein, wobei bei einer konischen Öffnung (253) die Spitze in Richtung des Behälterinnenraums (252) zeigt. Zumindest die zentral orientierte Oberfläche der Öffnung (253), die Innenwandung (321), weist eine höhere Oberflächenhärte auf als der Werkstoff des eingesetzten Stopfens (257), so dass der in die Öffnung (253) eingesetzte abdichtende Stopfen (257) elastisch verformt ist.

Die Behälteröffnung (253) kann Teil eines Verschlußeinsatzes (322) sein, vgl. Figur 15. Die Innenwandung (321) des z.B. aus Kunststoff hergestellten Verschlußeinsatzes (322) kann beispielsweise keramisiert sein.

Der Stopfen (257) hat an seiner z.B. zylinderförmigen Mantelfläche reifenförmige Wulste (324), mit denen er an der Innenwandung (321) abdichtend anliegt. Der durchbruchfreie Stopfen (257) hat an seiner Oberseite einen z.B. kegelförmigen Aufsatz (325), der im dargestellten Ausführungsbeispiel einen umlaufenden Ringwulst (326) aufweist.

Gegebenenfalls kann in den Darstellungen der Figuren 1 und 7 der Behälter (250) oberhalb des Stopfens (257) zusatzlich mittels einer Membran steril verschlossen sein.

Das Glasrohr (251) ist rückseitig mit einem beweglichen Kolben (261) verschlossen. Der Kolben (261) besteht aus einer Kolbenstange (262), einer hinteren Kolbendruckplatte (264), einem vorderen Stopfenträger (263) und einem darüber gestülpten elastischen Kolbenstopfen (267). Um den Kolben (261) bei einem vakuumisierten Zylinderinnenraum (252) in seiner hinteren Position zu halten, hat der Kolben (261) zusätzlich zwei oder mehrere Rastelemente (265), die z.B. an der Kolbendruckplatte (264) angeformt sind und sich - elastisch nach außen federnd - auf dem hinteren Rand des Glasrohres (251) abstützen. An der Rückseite des Kolbenstopfens (267) sitzt ein elastischer Gummiring (268), der die Rastelemente (265) nach außen drückt. Auf seiner Vorderseite hat der Kolbenstopfen (267) eine z.B. zylinderförmige Ausnehmung (269).

Die Kolbendruckplatte (264) hat zum Glasrohr (251) hin einen zylindrischen Bund (266), der den gleichen Außendurchmesser hat wie der Behälterbereich (221).

Zur Sicherung vor dem Auslösen ist der Behälteradapter (200) über die Banderole (90) mit dem Auslöseelement (82) des Injektors verbunden. Die Banderole (90) ist ein als Klebeetikett ausgebildeter Originalitätsverschluss.

Die Banderole (90) selbst ist z.B. ein mit einem Klebstoff bereichsweise einseitig beschichteter Papier- und/oder Folienstreifen. Sie besteht beispielsweise aus drei separaten Streifen, die jeweils über eine Perforation (96) oder über eine andere Sollbruchstelle gegeneinander abtrennbar sind. Die jeweils umlaufenden Perforationen (96) liegen oberhalb der Rillen (57) und unterhalb der Fenster (206).

Nach Figur 1 ist der Behälterbereich (221) und der Kolben (261) mit einer Abwickelbanderole (280) überklebt. Die Abwickelbanderole (280) überdeckt hierbei schützend die Fenster (226) und die Rastelemente (265) des Kolbens (261). Zusätzlich verhindert die Abwickelfolie (280) ein unbeabsichtigtes Herausziehen des Behälteradapters (200) aus dem Gehäuse (10).

Bei der Herstellung werden die beiden Zylinder-Kolben-Einheiten (100, 250) beispielsweise in getrennten Fertigungsverfahren hergestellt und auf unterschiedlichen Fertigungsstraßen befüllt. Der Behälteradapter (200) wird z.B. separat hergestellt. Die einzelnen Teile können somit auf Vorrat gefertigt werden und erst später mit dem Einweginjektor zusammengefügt werden. Die Komponenten (1 - 3) sind steril und können steril gelagert werden. Alle Teile, die mit Wirkstoffen und/oder Lösemittel in Kontakt kommen können, sind z.B. steril verpackt.

Zur Konfektionierung - vor der Auslieferung an den Anwender - wird beispielsweise die erste Zylinder-Kolben-Einheit (100) in den Einweginjektor (4) eingesetzt und verrastet. Auch der Behälteradapter (200) wird in den Einweginjektor (4) eingesetzt. Hierbei verbleibt z.B. die Membrankappe (290) auf dem Übertragungsrohr (242). Die zweite Zylinder-Kolben-Einheit (250) wird in den Behälterbereich (221) eingesteckt und in den dem Zwischenboden (211) abgewandten Rasthaken (223) verrastet. Der Stopfen (257) verschließt die Behälteröffnung (253) und berührt nicht das Übertragungsrohr (242).

Um den Einweginjektor benutzen zu können, muss der in der Zylinder-Kolben-Einheit (250) gelagerte Wirkstoff (2), z.B. ein Lyophilisat, in der im Zylinder (101) der Zylinder-Kolben-Einheit (100) vorhandenen Flüssigkeit (1), z.B. Wasser für Injektionszwecke, bzw. Physiologische Kochsalzlösung, gelöst werden. Dazu soll die Flüssigkeit (1) in den Behälter (250) gepumpt werden.

In einem ersten Schritt wird die Abwickelbanderole (280) vom Behälterbereich (221) entfernt und der Behälter (250) in der Behältereinschubrichtung (7) in den Behälteradapter (200) hineingeschoben, vgl. Figur 2. Die Rastelemente (223) werden nach außen verdrängt. Hierbei durchstößt das Rohr (242) z.B. die Membrankappe (290) und gegebenenfalls die Verschlußmembran des Behälters (250) und taucht in die Öffnung (253) ein. Beim weiteren Hineinschieben springt der z.B. kegelförmig ausgebildete Rastring (248) über den Ringwulst (326), wobei der Ringwulst (326) elastisch verformt wird. Nach dem Verrasten des Rastrings (248) verformt sich der Ringwulst (326) zurück und nimmt mit axialem und radialem Spiel seine Ausgangsgestalt oberhalb der z.B. waagerechten Rastnase (249) an, vgl. Figur 8.

Die in der Längsrichtung (5) orientierte axiale Länge des Rastrings (248) ist in diesem Ausführungsbeispiel kürzer als die axiale Länge des Stopfenaufsatzes (325), so dass beim Einschieben des Behälters (250) der Stopfen (257) mit seiner Stirnfläche (327) die Schiebefläche (247) kontaktiert. Der Stopfen (257) wird nun mittels des mit ihm verrasteten Übertragungsrohrs (242) in Richtung des Behälterinnenraums (252) verschoben. Das Übertragungsrohr (242) legt sich unter elastischer Verformung der Ringe (246) abdichtend in der Öffnung (253) an.

Die z.B. manuell betätigte Schubbewegung des Behälters (250) ist beendet, wenn der Stopfen (257) an den Anschlägen (225) anliegt. Die Einkerbungen (259) rasten in den Rastelementen (224) ein. Die Membrankappe (290) wird entlang des Übertragungsrohrs (242) verschoben. Beim Einschieben des Behälters (250) entweicht die hierbei verdrängte Luft durch die mit dem Ventilschlauch (229) steril abgedeckten Schieberausnehmungen (228) des Behälterbereichs (221). Der Stopfen (257) ist in der Darstellung der Figuren 3 und 9 in den Behälterinnenraum (252) verdrängt und hängt verliersicher am Übertragungsrohr (242). Die Querbohrung (245) steht in Richtung des Behälterinnenraums (252) aus der Öffnung (253) heraus.

Nach dem Eindringen des Übertragungsrohrs (242) in den Zylinderinnenraum (252) kommuniziert dieser über das Verbindungsrohr (242) mit dem Zylinderinnenraum (110) der ersten Zylinder-Kolben-Einheit (100). Das Vakuum des Zylinderinnenraums (252) saugt die Flüssigkeit aus dem Zylinder (101) der Zylinder-Kolben-Einheit (100). Da die rückseitige Abdeckung des Zylinders (101) eine Sterilfiltermembrane (119) ist, kann der angesaugte Kolben (111) der Flüssigkeit (1) folgen und kommt am Zylinderboden (108) zur Anlage. Im Innenraum (252) löst sich das Lyophilisat (2) in der Flüssigkeit (1). Der Lösevorgang kann über die Fenster (226) beobachtet werden.

In einem zweiten Schritt wird, sobald das Lyophilisat (2) gelöst ist, die Abreißbanderole (94) entfernt. Die Rillen (57) des Auslöseelements (82) werden damit sichtbar. Nun wird der Injektor so positioniert, dass die Zylinder-Kolben-Einheit (100) unterhalb der Zylinder-Kolben-Einheit (250) liegt. Danach soll die neu entstandene Lösung (3) durch das Übertragungsrohr (242) hindurch in den Zylinderinnenraum (110) gepumpt werden. Dazu wird der Kolben (261) durch ein radiales Eindrücken der Rastelemente (265) zunächst entsichert. Aufgrund des Restvakuums legt sich der Kolbenstopfen (267) auf die Oberfläche der Lösung (3). Nun wird durch leichten Druck auf den Kolben (261) die Lösung (3) in den Zylinderinnenraum (110) umgepumpt. Der Kolbenstopfen (267) wird in Richtung der Öffnung (253) verschoben. Hierbei umgreift er den Stopfen (257), den er z.B. in der Aussparung (269) aufnimmt. Die Lösung (3) schiebt den Kolben (111) vor sich her. Ein blasenfreies Befüllen des Zylinderinnenraumes (110) wird über die Fenster (206) im Durchlicht geprüft. In der Regel wird ein kleiner Teil der Lösung (3) in das Glasrohr (251) zurückgesaugt, so dass zudem der Kolben (111) nicht an der Sterilfiltermembrane (119) anliegt.

In einem dritten Schritt wird der Behälteradapter (200) mit der Zylinder-Kolben-Einheit (250) vom Gehäuse (10) abgezogen. Der Injektor (4) bleibt trotzdem gesichert, vgl. Figur 5.

Nach dem Aufsetzen des Injektors (4) mit der Zylinder-Kolben-Einheit (100) auf die desinfizierte Injektionsstelle muss in einem letzten Schritt, z.B. mit dem Daumen der den Injektor (4) haltenden Hand, der Sperrerknopf (132) gedrückt werden, um das Auslöseelement (82) zusammen mit der Auslösekappe (81) bewegen zu können. Nun kann das Auslöseelement (82) in Richtung der Zylinder-Kolben-Einheit (100) verschoben werden. Bei diesem Vorgang gleitet das Auslöseelement (82) auf der Außenwandung (13) des Gehäuses (10) linear nach unten, also in Richtung der Injektionsstelle. Die Anlageflächen (24) der Druckstäbe (21) rutschen über die Kante (85) und springen unter der Kraft des Federelements (50) entsichernd radial nach außen in die Aufweitung (83). Die Druckstäbe (21) haben sich elastisch nach außen gebogen und befinden sich nun in ihrer eigentlichen Ausgangslage. Die nun nicht mehr verformten Druckstäbe (21) geben den Kolbenbetätigungsstempel (60) frei, so dass sich der Kolbenschieber (76) unter der Wirkung des Federelements (50) ruckartig auf die Sterilfiltermembrane (119) des Zylinders (101) zubewegt. Die Sterilfiltermembrane (119) wird durchschlagen und der Kolben (111) zum Entleeren des Zylinders (101) nach unten bewegt, vgl. Figur 6. Der Zylinder (100) wird entleert.

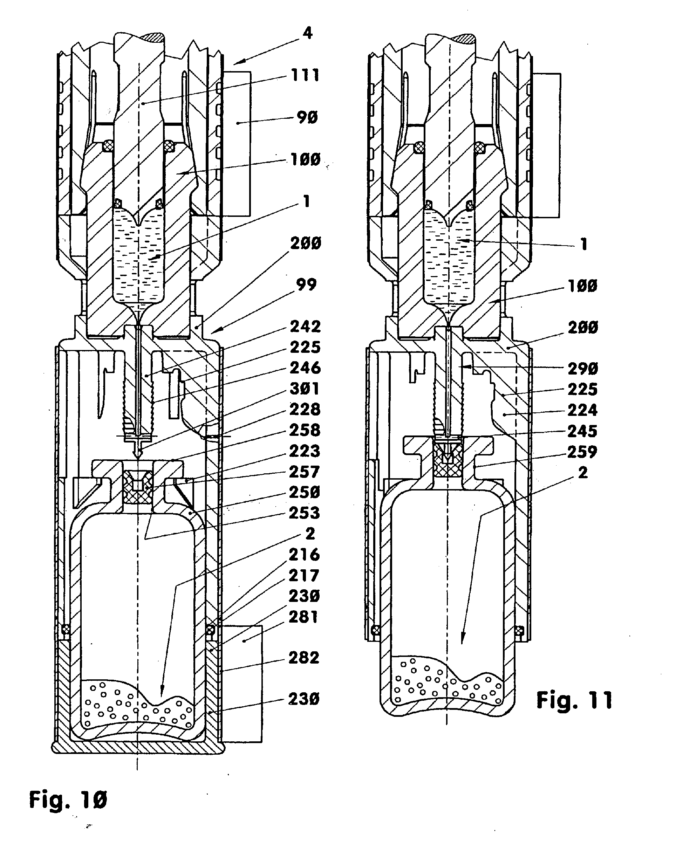

Die Figuren 10 bis 13 zeigen einen Einweginjektors (4) mit einem eingebauten Zwei-Kammer-System (99), dessen zweite Kammer (255) einen Behälter (250) mit einem konstanten Behältervolumen aufweist.

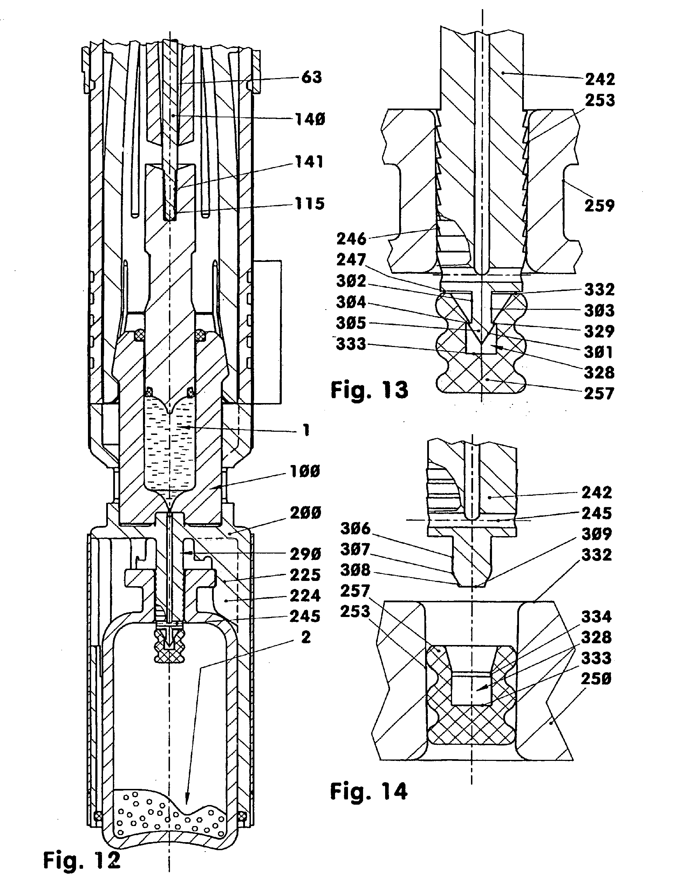

Der Einweginjektor (4) ist ähnlich aufgebaut wie der in den Figuren 1 - 6 dargestellte Einweginjektor (4). Der Kolben (111) hat jedoch an seiner Rückseite eine z.B. zentrale, kegelstumpfmantelförmige Ausnehmung (115), in der eine Pumpstange (140) mittels eines Kegelgewindes (141) eingeschraubt ist, vgl. Figur 12. Der Kolbenbetätigungsstempel (60) hat eine z.B. zentrale Bohrung (63), die von der Pumpstange (140) mit großem Spiel durchquert wird. Die aus dem Einweginjektor (4) herausragende Pumpstange (140) kann mit geringem Kraftaufwand vom Kolben (111) gelöst werden.

In den Behälteradapter (200) ist ein Übertragungsrohr (242) integriert, dessen Hauptabmessungen beispielsweise den Hauptabmessungen des im Zusammenhang mit dem ersten Ausführungsbeispiel beschriebenen Übertragungsrohrs (242) mit Querbohrung (245) entsprechen. Die Mantelfläche (303) des Übertragungsrohrs (242) trägt sich von unten nach oben konisch aufweitende, elastisch verformbare Ringe (246), deren maximaler Außendurchmesser größer ist als der Öffnungsdurchmesser des Behälters (250). Das Übertragungsrohr (242) kann bereichsweise verformbar sein. Es kann z.B. aus einem Zwei-Komponenten-Werkstoff mit einem verformungssteifen Kern und einem weichen, elastisch verformbaren Mantel bestehen.

Der Behälteradapter (200) hat zwei z.B. mittels eines Ventilschlauchs (229) verschlossene Schieberdurchbrüche (228), die mit der Querbohrung (245) fluchten.

Das Rohrende (243) des Übertragungsrohrs (242) ist pfeilförmig ausgebildet und hat eine den Pfeil (301) umgebende Schiebefläche (247).

Der Spitzenwinkel des Pfeils (301) beträgt beispielsweise 60 Grad. Die hier nach oben gerichtete Rückseite (304) der Pfeilspitze (305) umfasst eine ringförmige Ebene, die normal zur Richtung des Übertragungsrohrs (242) ausgerichtet ist.

Der in der Behälteröffnung (253) sitzende Stopfen (257) hat an seiner Oberseite eine zentrale, als Sackloch ausgebildete Ausnehmung (328). Die Tiefe dieser Ausnehmung (328) ist größer als die Länge des Pfeils (301) einschließlich des Schafts (302). Unterhalb eines Absatzes (329) ist die Ausnehmung (328) beispielsweise zylinderförmig ausgebildet. Der Durchmesser dieses Bereichs ist größer als der maximale Durchmesser der Pfeilspitze (305), seine Tiefe ist größer als die Länge der Pfeilspitze (305).

Oberhalb des Absatzes (329) - der Durchmesser des Absatzes (329) ist größer als der Durchmesser des Pfeilschafts (302) und kleiner als der größte Durchmesser der Pfeilspitze (305) - ist die Ausnehmung (328) z.B. kegelabschnittsförmig ausgebildet. Sie hat hier einen Öffnungswinkel von beispielsweise 60 Grad, wobei die gedachte Kegelspitze in Richtung des Behälterinnenraums (252) orientiert ist.

Der Behälter (250) ist z.B. ein Glasfläschchen, bzw. ein Lyophilisatfläschchen, mit einem taillierten Hals (259) und einem Flanschrand (258). Der Flanschrand (258) steht über den Hals (259) über. Sein Außendurchmesser ist jedoch kleiner als der maximale Behälteraußendurchmesser. Der Übergang zwischen dem Hals (259) und der zylindrischen Außenwandung des Behälters (250) ist mit einem großen Radius abgerundet, der z.B. der doppelten Behälterwandstärke entspricht. Der Behälter (250) wird über eine Kappe (230) und eine Abreißbanderole (280) am Behälteradapter (200) gesichert.

Um den Einweginjektor (4) benutzen zu können, muss der im Behälter (250) gelagerte Wirkstoff (2), z.B. das Lyophilisat, in der im Zylinder (101) der Zylinder-Kolben-Einheit (100) vorhandenen Flüssigkeit (1), z.B. Wasser für Injektionszwecke, bzw. Physiologische Kochsalzlösung, gelöst werden. Dazu soll die Flüssigkeit (1) in den Behälter (250) gepumpt werden.

In einem ersten Schritt wird die Abreißfahne (281) von der Kappe (230) unter einem Auftrennen der Perforation (282) entfernt und die Kappe (230) vom hinteren Teil des Behälters (250) abgezogen.

In der Figur 10 ist der Behälter mittels des durchbruchfreien Stopfens (257) verschlossen. Gegebenenfalls können sowohl der Behälter (250) als auch die Öffnungen der Querbohrung (245) des Verbindungsrohrs (242) zusätzlich mittels einer Membran bzw. mittels einer Membrankappe verschlossen sein.

Ggf. befindet sich in einer Ringnut (216) des Behälterbereichs (221) ein elastischer Dichtring (217), der die Fuge zwischen dem Behälter (250) und der Innenwandung des Behälterbereiches (221) steril verschließt.

In einem zweiten Schritt wird der Behälter (250) in den Behälteradapter (200) hineingeschoben. Dabei gleitet der Behälter (250) an der Innenwandung des Behälteradapters (200) nach vorn, bis er mit dem Flanschrand (258) an den Anschlägen (225) anliegt, vgl. die Figuren 11 und 12. Gleichzeitig umgreifen die Rasthintergriffe (224) die Rückseite des Flanschrandes (258) und sichern so die vordere Position des Behälters (250). Bei der Vorwärtsbewegung hat der Behälter (250) die Klapprasthaken (223) zur Seite gedrückt und die Rasthaken (224) verrasten den Behälter (250). Die Figur 12 zeigt einen Querschnitt des Behälteradapters (200) und des eingesetzten Behälters (250).

Nach den Ausführungsbeispielen der Figuren 10 und 11 taucht das Übertragungsrohr (242) beim Einschieben des Behälters (250) in die Öffnung (253) und in die Ausnehmung (328) des Stopfens (257) ein. Hierbei verformt die Pfeilspitze (305) den Absatz (329) der Ausnehmung (328) elastisch und dringt in den hier unterhalb des Absatzes (329) dargestellten Bereich ein. Beim weiteren Eindringen der Pfeilspitze (305) verformt sich der Absatz (329) elastisch zurück, so dass sich eine den Pfeilschaft (302) umgebender z.B. konzentrischer Durchbruch verbleibt, der die Pfeilspitze (305) hintergreift. Der Stopfen (257) ist mit dem Behälteradapter (200) verrastet, vgl. Figur 13.

Das Übertragungsrohr (242) taucht in diesem Ausführungsbeispiel in die Ausnehmung (328) ein, bis die Schiebefläche (247) auf der Stopfenoberseite (332) aufsetzt. Die Pfeilspitze (305) berührt nicht den Boden (333) der Ausnehmung (328). Beim weiteren Einschieben des Behälters (250) verdrängt der Behälteradapter (200) mit dem Übertragungsrohr (242) den Stopfen (257) in den Behälterinnenraum (252). Das Übertragungsrohr (242) dichtet nun die Öffnung (253) vollständig ab, vgl. Die Figuren 12 und 13.

Sobald der Stopfen (257) die Öffnung (253) verlassen hat, rutscht er entlang des Pfeilschafts (302) in der Darstellung der Figur 13 nach unten, bis die Unterseite des Absatzes (329) auf der Rückseite (304) der Pfeilspitze (305) aufliegt. In dieser Bewegungsrichtung ist der Verformungswiderstand des Absatzes (329) so groß, dass die Schwerkraft des Stopfens (257) nur eine unwesentliche Verformung des Absatzes (329) bewirkt. Der Stopfen (257) hängt unlösbar an der Pfeilspitze (305), die sein Herunterfallen verhindert. Die Querbohrung (245) ragt in den Zylinderinnenraum (252) und liegt beispielsweise im Anschlußbereich des Flaschenhalses (259), so dass sie gerade eben frei ist.

Der Zylinderinnenraum (110) und der Behälterinnenraum (252) kommunizieren über die Querbohrung (245) und die Bohrung (244) des Übertragungsrohrs (242). Die Rasthaken (224) verhindern ein Herausziehen des Behälters (250).

Der beim Einschieben des Behälters (250) im Behälterbereich (221) entstehende Überdruck entweicht über die Schieberausnehmungen (228) unter z.B. partiellem Abheben des Ventilschlauchs (229), das auch den Innenraum steril hält. Die Schieberausnehmungen (228) und der Ventilschlauch (229) haben somit die Funktion eines Überdruckventils.

In einem dritten Schritt wird der Kolben (111) mittels der Pumpstange (140) in den Zylinder (101) geschoben und so die Flüssigkeit (1) in den nun unter leichtem Überdruck stehenden Behälterinnenraum (252) gefördert. Die Pumpstange (140) wird dazu in der Regel zwischen dem Zeigefinger und dem Daumen der bedienenden Hand feinfühlig gehalten.

Das Lyophilisat (2) löst sich in der Flüssigkeit (1). Der Lösevorgang kann optisch kontrolliert werden, da der aus dem Behälteradapter (200) herausragende Behälter (250) transparent ist.

In einem vierten Schritt wird die neu entstandene Lösung (3) in den Zylinderinnenraum (110) zurückgepumpt. Dazu wird der Injektor so gehalten, dass die Öffnung (253) des Behälters (250) in Schwerkraftrichtung zeigt. Der Kolben (111) wird über die Pumpstange (140) in eine hintere Position gezogen. Ein blasenfreies Befüllen wird über die Fenster (206) geprüft. Durch die Lage des Querrohrs (245) ist eine weitgehende Entleerung des Behälters (250) gewährleistet.

In einem fünften Schritt wird zum Entsichern des Einweg-Injektors die Abreißbanderole (94) mit Hilfe der Abreißfahne (95) ringsherum vom Hauptteil (92) und vom Adapterteil (93) getrennt. Die Rillen (57) des Auslöseelements (82) werden sichtbar. Der Behälteradapter (200) wird nun z.B. nach unten vom Zylinder (101) abgezogen.

In einem letzten Schritt wird der Injektor auf die desinfizierte Injektionsstelle gesetzt und das hülsenartige Auslöseelement (82) nach unten - in Richtung der Injektionsstelle - geschoben. Die Druckstäbe (21) biegen sich elastisch nach außen in ihre eigentliche Ausgangslage. Hierbei rutschen die Nocken (22) über die Kante (85) nach außen in die Aufweitung (83). Die nun nicht mehr verformten Druckstäbe (21) geben den Kolbenbetätigungsstempel (60) frei, so dass sich der Kolben (111) unter der Wirkung des Federelements (50) ruckartig zum Entleeren des Zylinders (101) nach unten bewegt. Bei der Vorwärtsbewegung des Kolbens (111) vermindert sich die Kolbenreibung zwischenzeitlich, da das rückwärtige Dichtelement beim Passieren des taillierten Kolbenbereiches nicht bremsend anliegt.

Die Figur 14 zeigt eine Variante der Kopplung zwischen dem Behälteradapter (200) und dem Stopfen (257). Die Pfeilspitze (305) ist in diesem Ausführungsbeispiel durch einen zylindrischen Schaft (306) ersetzt, der sich unterhalb eines Ringwulsts (307) kegelabschnittsförmig verjüngt. Dieser greift beim Verschieben des Behälters (250) z.B. in eine kalottenförmig ausgebildete Nut (334) der Stopfenausnehmung (328) ein. Im dargestellten Ausführungsbeispiel bildet die Stirnseite (309) des kegelabschnittsförmigen Bereichs (308) die Schiebefläche, mit der das Übertragungsrohr (242) den Stopfen (257) aus der Behälteröffnung (253) herausschiebt. Bei einem Behälteradapter (200) mit einem kürzer ausgebildeten Übertragungsrohr (242) ist die Schiebefläche (247) um das Übertragungsrohr (242) herum angeordnet.

Auch Kombinationen der beschriebenen Ausführungsbeispiele sind denkbar.

### Bezugszeichenliste:

- 1: Wasser für Injektionszwecke, Lösemittel
- 2: Lyophilisat, Wirkstoff, Arzneistoff
- 3: Injektionslösung
- 4: Einweginjektor
- 5: Mittellinie des Injektors, Längsrichtung
- 6: Auslösebewegungsrichtung von (82), Abwärtsbewegung Richtungspfeil
- 7: Behältereinschubrichtung

- 10: Gehäuse, einteilig
- 13: Außenfläche, zylindrisch, Außenwandung

- 21: Druckstäbe, Stützstäbe
- 22: Nocken
- 23: Abstützfläche
- 24: Anlagefläche
- 28: Biegebalken

- 31: Mantelbereich
- 33: Durchbrüche
- 39: Boden

- 41: Fixierbereich für die Zylinder-Kolben-Einheit
- 42: Federhaken
- 43: Hintergriff

- 50: Federelement, Schraubendruckfeder, Federenergiespeicher
- 57: Rillen von (82)
- 59: Innenwandung von (82)

- 60: Kolbenbetätigungsstempel
- 62: Führungszapfen
- 63: Bohrung
- 73: Stempelteller
- 75: Bundfläche, konisch
- 76: Kolbenschieber

- 80: Auslöseeinheit
- 81: Auslösekappe
- 82: Auslöseelement, Auslösehülse
- 83: Aufweitung
- 84: Rücksprungflanke
- 85: Kante, scharfkantig

- 90: Originalitätsverschluss, Banderole, Sicherungselement, Klebeetikett
- 92: Randteil, hinten; Etikettteil
- 93: Randteil, vorn; Etikettteil
- 94: Abreißbanderole
- 95: Abreißfahne
- 96: Perforationen, Sollbruchstellen

- 99: Zwei-Kammer-System
- 100: Zylinder-Kolben-Einheit, erste, injektorseitig
- 101: Zylinder, injektorseitig
- 102: Rastring
- 103: Stirnfläche
- 104: Klebering
- 105: Kammer
- 106: Bohrung, Düse
- 107: Ausnehmung in der Stirnfläche
- 108: Zylinderboden
- 110: Zylinderinnenraum
- 111: Kolben
- 112: Ringnut
- 114: Dichtring, Dichtung
- 115: Ausnehmung in (111)
- 119: Sterilfiltermembrane

- 132: Sperrerknopf

- 140: Pumpstange
- 141: Kegelgewinde

- 200: Behälteradapter
- 201: Adapterbereich
- 204: Absatz, ringförmig
- 206: Fenster, beidseitig
- 211: Zwischenboden
- 213: Erhebung
- 216: Ringnut
- 217: Dichtring

- 221: Behälterbereich, Behälteraufnahme
- 223: Rastelemente, Klapprasthaken
- 224: Rastelemente, Rasthintergriffe
- 225: Anschläge
- 226: Fenster
- 228: Schieberausnehmungen
- 229: Ventilschlauch

- 230: Kappe

- 242: Übertragungsrohr, Verbindungsrohr
- 243: Rohrende
- 244: Bohrung, Übertragungsrohrbohrung
- 245: Querbohrung
- 246: Ringe
- 247: Schiebefläche
- 248: Rastring
- 249: Rastnase

- 250: Zylinder-Kolben-Einheit, zweite, Behälter
- 251: Rohr, Glasrohr, Kunststoffrohr
- 252: Zylinderinnenraum, Behälterinnenraum
- 253: Öffnung
- 255: Kammer
- 257: Stopfen, elastisch, Gummistopfen
- 258: Flanschrand
- 259: Einkerbung, Hals

- 261: Kolben
- 262: Kolbenstange
- 263: Stopfenträger
- 264: Kolbendruckplatte
- 265: Rastelemente
- 266: Bund
- 267: Kolbenstopfen
- 268: Gummiring, Elastomerfeder
- 269: Ausnehmung, Aussparung

- 280: Abwickelbanderole
- 281: Abreißfahne
- 282: Perforation

- 290: Membrankappe
- 291: Wandbereich
- 292: Membran, Kappenmembran

- 301: Pfeil
- 302: Schaft
- 303: Mantelfläche
- 304: Rückseite
- 305: Spitze
- 306: zylindrischer Schaft
- 307: Ringwulst
- 308: kegelabschnittsförmiger Bereich
- 309: Stirnseite

- 321: Innenwandung
- 322: Verschlußeinsatz
- 324: Wulste
- 325: Aufsatz, Stopfenaufsatz
- 326: Ringwulst
- 327: Stirnfläche
- 328: Ausnehmung
- 329: Absatz

- 332: Stopfenoberseite
- 333: Boden von (328)
- 334: Nut

## Patentansprüche

1. Einweginjektor (4) und Zwei-Kammer-System (99), wobei zumindest eine erste Kammer (105) Teil einer im Einweginjektor (4) aufnehmbaren Zylinder-Kolben-Einheit (100) ist und wobei die zweite Kammer (255) Teil eines zumindest zeitweise mittels eines Stopfens (257) verschlossenen und in einem lösbar am Einweginjektor (4) gelagerten Behälteradapter (200) eingesetzten Behälters (250) mit mindestens einer Öffnung (253) ist, **dadurch gekennzeichnet,**
- **dass** der Stopfen (257) und der Behälteradapter (200) miteinander unlösbar verrastbar sind,
- **dass** beim Einsetzen des Behälters (250) der Behälteradapter (200) die Öffnung (253) unter Verdrängung des Stopfens (257) verschließt und
- **dass** der Behälteradapter (200) nach dem Einsetzen des Behälters (250) einen Zylinderinnenraum (110) der Zylinder-Kolben-Einheit (100) mit einem Innenraum (252) des Behälters (250) verbindet.

2. Einweginjektor (4) und Zwei-Kammer-System (99) nach Anspruch 1, **dadurch gekennzeichnet, dass** die Öffnung (253) des Behälters (250) zylindrisch oder kegelmantelförmig ausgebildet ist, wobei bei einer kegelmantelförmig Ausbildung die gedachte Kegelspitze im Behälterinnenraums (252) angeordnet ist.

3. Einweginjektor (4) und Zwei-Kammer-System (99) nach Anspruch 1, **dadurch gekennzeichnet, dass** der Behälteradapter (200) ein zumindest bereichsweise elastisch verformbares Übertragungsrohr (242) umfasst.

4. Einweginjektor (4) und Zwei-Kammer-System (99) nach Anspruch 3, **dadurch gekennzeichnet, dass** die Innenwandung (321) der Öffnung (253) härter ist als der Stopfen (257) und der verformbare Bereich des Übertragungsrohrs (242).

5. Einweginjektor (4) und Zwei-Kammer-System (99) nach Anspruch 3, **dadurch gekennzeichnet, dass** das Übertragungsrohr (242) mit dem Stopfen (257) verrastet.

6. Einweginjektor (4) und Zwei-Kammer-System (99) nach Anspruch 3, **dadurch gekennzeichnet, dass** die Übertragungsrohrbohrung (244) in eine das Übertragungsrohr (242) durchdringende Querbohrung (245) mündet.

7. Einweginjektor (4) und Zwei-Kammer-System (99) nach Anspruch 1, **dadurch gekennzeichnet, dass** der Behälteradapter (200) eine Schiebefläche (247) zum Verdrängen des Stopfens (257) aufweist.

## Claims

1. Single-use injector (4) and two-chamber system (99), at least a first chamber (105) being part of a cylinder/piston unit (100) which can be received in the single-use injector (4) and the second chamber (255) being part of a container (250) having at least one opening (253), which container is closed at least temporarily by means of a stopper (257) and inserted in a container adapter (200) which is detachably mounted on the single-use injector (4), **characterized**
- **in that** the stopper (257) and the container adapter (200) can be interlocked nondetachably to each other,
- **in that**, when the container (250) is inserted, the container adapter (200) closes the opening (253) by displacing the stopper (257), and
- **in that**, after the container (250) has been inserted, the container adapter (200) connects a cylinder interior (110) of the cylinder/piston unit (100) to an interior (252) of the container (250).

2. Single-use injector (4) and two-chamber system (99) according to Claim 1, **characterized in that** the opening (253) of the container (250) is formed cylindrically or in the shape of a conical envelope, with, in the case of a formation in the shape of a conical envelope, the notional cone apex being arranged in the container interior (252).

3. Single-use injector (4) and two-chamber system (99) according to Claim 1, **characterized in that** the container adapter (200) comprises a transfer tube (242) which is elastically deformable at least in certain regions.

4. Single-use injector (4) and two-chamber system (99) according to Claim 3, **characterized in that** the inner wall (321) of the opening (253) is harder than the stopper (257) and the deformable region of the transfer tube (242).

5. Single-use injector (4) and two-chamber system (99) according to Claim 3, **characterized in that** the transfer tube (242) interlocks with the stopper (257).

6. Single-use injector (4) and two-chamber system (99) according to Claim 3, **characterized in that** the transfer tube hole (244) opens into a transverse hole (245) penetrating the transfer tube (242).

7. Single-use injector (4) and two-chamber system (99) according to Claim 1, **characterized in that** the container adapter (200) has a sliding surface (247) for displacing the stopper (257).

## Revendications

1. Dispositif d'injection à usage unique (4) et système à deux chambres (99), au moins une première chambre (105) faisant partie d'une unité de cylindre-piston (100) pouvant être reçue dans le dispositif d'injection à usage unique (4), et la deuxième chambre (255) faisant partie d'un récipient (250) doté d'au moins une ouverture (253), fermé au moins temporairement au moyen d'un bouchon (257) et inséré dans un adaptateur de récipient (200) monté de manière amovible sur le dispositif d'injection à usage unique (4), **caractérisés**
- **en ce que** le bouchon (257) et l'adaptateur de récipient (200) peuvent être encliquetés l'un avec l'autre de manière non amovible,
- **en ce que**, lors de l'insertion du récipient (250), l'adaptateur de récipient (200) ferme l'ouverture (253) en déplaçant le bouchon (257) et
- **en ce qu'**après l'insertion du récipient (250), l'adaptateur de récipient (200) relie un espace intérieur de cylindre (110) de l'unité cylindre-piston (100) à un espace intérieur (252) du récipient (250).

2. Dispositif d'injection à usage unique (4) et système à deux chambres (99) selon la revendication 1, **caractérisés en ce que** l'ouverture (253) du récipient (250) est réalisée de manière cylindrique ou sous forme d'enveloppe conique, le sommet de cône imaginaire étant disposé dans l'espace intérieur de récipient (252) en cas de réalisation sous forme d'enveloppe conique.

3. Dispositif d'injection à usage unique (4) et système à deux chambres (99) selon la revendication 1, **caractérisés en ce que** l'adaptateur de récipient (200) comporte un tube de transfert (242) déformable élastiquement au moins dans certaines régions.

4. Dispositif d'injection à usage unique (4) et système à deux chambres (99) selon la revendication 3, **caractérisés en ce que** la paroi intérieure (321) de l'ouverture (253) est plus dure que le bouchon (257) et que la région déformable du tube de transfert (242).

5. Dispositif d'injection à usage unique (4) et système à deux chambres (99) selon la revendication 3, **caractérisés en ce que** le tube de transfert (242) est encliqueté avec le bouchon (257).

6. Dispositif d'injection à usage unique (4) et système à deux chambres (99) selon la revendication 3, **caractérisés en ce que** le tube de transfert (244) débouche dans un alésage transversal (245) traversant le tube de transfert (242).

7. Dispositif d'injection à usage unique (4) et système à deux chambres (99) selon la revendication 1, **caractérisés en ce que** l'adaptateur de récipient (200) comprend une surface de glissement (247) pour déplacer le bouchon (257).
